Europäisches Patentamt

European Patent Office

Office européen des brevets

(19)

(11) Publication number: **0 173 663 B1**

# EUROPEAN PATENT SPECIFICATION

(12)

(45) Date of publication of patent specification: **02.01.92**

(51) Int. Cl.⁵: **G01N 33/577**, G01N 33/574, A61K 39/395, A61K 49/02, //C12P21/00,C12N15/00, C07H3/06

(21) Application number: **85850256.0**

(22) Date of filing: **06.08.85**

(54) **The use of a specific tumor associated antigen, sialosyllactotetraose, in diagnostic or therapeutic procedures related to cancer deseases.**

(30) Priority: **28.08.84 SE 8404283**

(43) Date of publication of application:
**05.03.86 Bulletin 86/10**

(45) Publication of the grant of the patent:
**02.01.92 Bulletin 92/01**

(84) Designated Contracting States:
**AT BE CH DE FR GB IT LI LU NL**

(56) References cited:
**EP-A- 0 118 365**
**WO-A-84/04599**
**US-A- 4 471 057**

(73) Proprietor: **Kabi Pharmacia AB**

**S-751 82 Uppsala(SE)**

(72) Inventor: **Holmgren, Jan Roland**
**Stötekärrsvägen 11 D**
**S-421 77 Västra Frölunda(SE)**
Inventor: **Lindholm, Leif Gustav**
**Postlada 5603**
**S-430 31 Kullavik(SE)**
Inventor: **Svennerholm, Lars Torvald**
**Fotbollsgatan 9**
**S-431 39 Mölndal(SE)**

(74) Representative: **Widén, Björn et al**
**Kabi Pharmacia AB**
**S-751 82 Uppsala(SE)**

EP 0 173 663 B1

CHEMICAL ABSTRACTS, vol. 101, no. 23, 3rd December 1984, page 464, abstract no. 208794y, Columbus, Ohio, US; Y. FUKUSHI et al.: "Novel fucolipids accumulating in human adenocarcinoma, III. A hybridoma antibody (FH6) defining a human cancer-associated difucoganglioside (VI3NeuAcV3III3Fuc2nLc6)", & J. BIOL. CHEM. 1984, 259(16), 10511-17

CHEMICAL ABSTRACTS, vol. 98, no. 11, 14th March 1983, page 396, abstract no. 87372t, Columbus, Ohio, US; L.C. HUANG et al.: "Many monoclonal antibodies with an apparent specificity for certain lung cancers are directed against a sugar sequence found in lacto-N-fucopentaose III.", & ARCH. BIOCHEM. BIOPHYS, 1983, 220(1), 318-20

CHEMICAL ABSTRACTS, vol. 103, no. 7, 19th August 1985, page 402, abstract no. 52062m, Columbus, Ohio, US; O. NILSSON et al.: "Fucosyl-Gm1 - a ganglioside associated with small cell lung carcinomas", & GLYCOCONJUGATE J. 1984, 1(1), 43-9

Int. Archs. Allergy appl. Immun. 71: 178-181 (1983); Lindholm et al: "Monoclonal Antibodies against Gastrointestinal Tumour-Associated Antigens Isolated as monosialogangliosides"

Biochemica et Biophysica Acta 834 (1985) 110-117; Manson et al: "Chemical structure of carcinoma ganglioside antigens defined by monoclonal antibody C-50 and some allied gangliosides of human pancreactic adenocarcinoma"

British Medical Journal, 19 May 1984, 288, 1479-1482; Holmgren, Lindholm et al: Detection by monoclonal antibody of carbohydrate antigen CA 50 in serum of patients with carcinoma"

Glycoconjugates. Proceedings of 7th International Symposium, Ed. Chester MA et al. Lund University, Lund, Symposium Secretariat (1982), 71, 852-3; Nilsson et al: "Tissue Distribution and concentration of a monoclonal antibody defined Tumour-Associated Ganglioside Antigen"

Journal of Biological Chemistry, 257 (1982) 14365-9; Magnani et al: "A monoclonal antibody-defined antigen associated with gastrointestinal cancer is a ganglioside containing sialylated lacto-n-fucopentaose II"

Biochem. Biophys. Res. commun. 110 (1983) 383-91; Falf et al: "MASS SPECTOMETRY OF A HUMAN TUMOR GLYCOLIPID ANTIGEN BEING DEFINED BY MOUSE MONOCLONAL ANTIBODY NS-19-9"

## Description

### Field of the invention

This invention relates to the use of a specific tumor associated antigen of carbohydrate nature, sialosyllactotetraose-IV$^3$NeuAcLcOse$_4$-(IUPAC-IUB Lipid Document, 1977) for diagnostic and therapeutic procedures related to human tumor deseases.

### Background

It is established that the transformation of tissue cells to tumor cells is associated with a change of the carbohydrate structure on the cell surface. Many carbohydrate structures serve as antigens and the tumor-modified structures represent a type of so-called tumor-associated antigens. The cell surface carbohydrate structures are linked either to a lipid moiety, in which case they are called glycolipids, or to proteins (peptides), in which case they are called glycoproteins (glycopeptides). A common designation for the two forms is glycoconjugate.

Tumor-associated glycoconjugate antigens have previously been known in relation to human tumor diseases. In melanomas the carbohydrate structures sialosylgangliotetraose and sialosyllactose have been identified linked to a lipid portion. Two carcinoma associated antigens CEA (carcino-embryonal antigen) and GICA (gastrointestinal cancer antigen) have been demonstrated particularly in gastrointestinal carcinomas while a third antigen, CA-50, seems to be a general carcinoma antigen. All these antigens are secreted from the tumor cell surface and cube demonstrated in blood serum.

The present invention is based on the surprising discovery that patients with many forms of carcinoma express in the primary tumor and its metastases a glycoconjugate, which is not expressed in normal tissue.

In accordance with the invention the glycoconjugate antigen has been isolated as a glycolipid antigen. It is a ganglioside having the complete chemical structure NeuAcα-3Galβ-3GlcNAcβ-3Galβ-4Glc-Cer or NeuAc LcOse$_4$Cer (IUPAC-IUB Lipid Document, 1977). A glycolipid having the basic strucure of the present ganglioside but with no sialic acid has been demonstrated in i.a. human meconium but in spite of extensive investigations it has not been possible to demonstrate the present ganglioside in any human tissue from an adult. However, it has been possible to find said ganglioside in lipid extracts in many carcinomas in different organs. Within the scope of the present invention sensitive procedures have been elaborated for the detection of said antigen in body fluids and tissues. All the procedures are based on the fact that the antigen which is present in very low concentrations is determined by binding to a specific monoclonal antibody. Several monoclonal antibodies have been prepared upon immunization of mice with a human colon carcinoma cell line. Said monoclonal antibodies define sialosyllactotetraose when it is present as a monosialo ganglioside, i.e. a carbohydrate - containing lipid which contains a sialic acid as a characteristic component but it also defines all those antigens where the same carbohydrate structure is bonded to a protein. The sialic acid is necessary for the antigen activity since hydrolysis of the antigen with sialidase enzyme completely removes the ability of the antigen to bind to the monoclonal antibodies. The monoclonal antibodies also define antigens which, in addition to sialosyllactotetraose, contain a fucose moiety linked to the molecule.

Within the scope of the present invention procedures have been elaborated for the isolation of the tumor associated glycolipid, sialosyllactotetraosylceramide, in pure form from human tumor tissue. The purified ganglioside has by a special process obtained an increased immunogenic activity and has been used for the production of monoclonal antibodies. Procedures have, within the scope of the invention, been developed for the indentification and determination of said antigen in tissues, tissue sections, and body fluids. All of these methods for diagnostic purposes are based on the fact that the antigen can be detected by binding to the specific monoclonal antibodies produced against the pure ganglioside antigen, sialosyllactotetraosylceramid. The following examples illustrate the various sources where the ganglioside antigen can be measured: surgical or autopsy material of tumor tissue from lung, liver, intestine, stomach or pancreas as well as cells obtained by biopsy of tumor tissue in different organs, by aspirations of the stomach or respiratory tract, expectorations, or by puncture of pleural or abdominal cavity, and biological fluids like blood, blood plasma, serum, lymph fluid, cerebrospinal fluid, urine, saliva or similar.

The antigen which is used according to the present invention is termed, sialosyllactotetraose and has the following structure: NeuAcα-3Galβ-3Glc-NAcβ-3Galβ-4Glc or IV$^3$NeuAcLcOse$_4$ -(IUPAC-IUB Lipid Document, 1977). Within the scope of the present invention all antigens fall, which have been modified by linking fucose to the N-acetylglucosamine molecule in a α-4 bond. The antigens used to define the monoclonal antibodies have been produced from antigens bound to a lipid group but also includes the binding to every other compound.

The antigen used according to the invention can be isolated from tumor tissue, obtained by autopsy of patients. A particularly good source are certain forms of lung carcinoma. The concentration of the antigen in the tumor tissue is in general low, and the isolation of the antigen will then be laborious. We have found another biological starting material for the isolation of the tumor antigen.

The cell line Colo 205 results in tumors when inplanted to so-called nude mice (generic name nu/nu). These tumors contain a high concentration of the antigen to which also fucose is linked. By treating the isolated ganglioside with the α-fucosidase enzyme the fucose moiety may be split off and the glycolipid antigen be obtained in pure form.

The present invention concerns in accordance herewith the induction of the tumor antigen with chromatographic methods from tumors induced by Colo 205 cells on so-called nude mice or from human tumors. The present invention also relates to the production of monoclonal antibodies against the purified antigen adsorbed to a certain bacterial membrane. The invention includes also the use of the chemical compound sialosyllactotetraose, all the derivates of this substance and all types of antibodies directed against them for the diagnosis of different cancer forms in different organs, and for the treatment of cancer diseases in patients.

Example 1

PREPARATION OF SIALOSYLLACTOTETRAOSE ANTIGEN IN THE FORM OF GANGLIOSIDE FROM SO-CALLED NUDE MICE AS WELL AS FROM HUMAN CANCER TISSUE.

One million viable COLO 205 cells suspended in 0,1 ml of 0,85 % NaCl. 1/15 M phosphate buffert, pH 7,2, were inocculated subcutaneously on 4 locations on each mouse. After 4 to 5 weeks, depending on the size of the tumors grown, the animal was sacrificed and the tumors were cut away and homogenized.

Extraction of gangliosides from tissue

The tissue was homogenized after the addition of 3 volumes of water to one volume of tissue material. After homogenization of the tumor tissue with a knife homogenizer at 1500 rpm during 3 min., 10 volumes of methanol were added under stirring and 5 volumes of chloroform. A clear extract was isolated by centrifugation. The tissue lump 4 volumes of water were added and the tissue was homogenized again and 10 volumes of methanol and 5 volumes of chloroform were added under stirring. A clear extract was separated by centrifugation. To the combined extracts water was added to give a final chloroform-methanol-water ratio of 4:8:5.6 (by volume). After mixing in a separatory funnel, two upper phase was separated. To the lower phase there was added on equally large volume of methanol and upon mixing 0,7 volumes of water. After careful mixing the separation funnel was set aside until two distinct liquid phases were obtained. The upper phase was collected and combined with the first one. To the combined upper phases there was added isobutanol to prevent foaming, and then the extract was evaporated in a rotaratory evaporator on a water bath having a temperature of 50°C. To the residue there was added 0,5 volume of methanol per volume of original tissue and 0,5 volumes of 1,0 M potassium hydroxide and was left overnight at room temperature. Then there was added 1,0 M hydrochloric acid in water to pH 8 under vigorous mixing and then against water was carried out for 48 hours.

The same extraction as for tumors on nude mice was carried out with human tumor tissue.

Isolation of a monosialoganglioside fraction by ion exchange chromatography

The dialysed crude ganglioside extract was evaporated and redissolved in 1 ml of chloroform-methanol-water (60:30:4.5) per gram of original tissue. A column with an inner diameter of 20 mm was packed with Spherosil-DEAE-dextran. (Institute Meriéux, Lyo, France) in acetate form in the same solvent -at least 1 ml of ion exchanger per each 5 g of tissue. The pure ganglioside extract was added slowly to the column and the column was then eluted with 10 bed volumes of chloroform-methanol-water (60:30: 4.5). The monosialoganglioside fraction was then eluted with 10 bed volumes of 0,02 M potassium acetate in methanol. The monosialoganglioside fraction was evaporated and desalted by dialysis against distilled water.

Purification of sialosyllactotetraosylceramid by silica gel chromatography

For the chromatography 1 g of latrobeads (Latron Laboratories Inc., Tokyo, Japan) was used for each g of original tissue, but at least 25 g. The gel was slurried in chloroform-methanol (volume ratio 4:1) and poured into a glass column provided with a sintered glass filter and having a diameter of 15-20 mm depending on the amount of gel. The ganglioside fraction was dissolved in 10 ml of chloroform-methanol-water (65:25:4, by volume) and applied to the column. It was eluted first with 15 ml of chloroform-methanol-water (65:25:4) for each g (weight/volume) of gel, which was collected in one fraction. The elution was then continued with chloroform-methanol-2.5 M ammonia (60:40:9, by volume) and 5 ml fractions were sampled on a fraction collector. The elution was monitored by testing 10 $\mu$l of each fraction by high performance thin-layer chromatography with the same solvents as used for the column chromatography (HPTLC) with the same solvent as used in the column chromatography, viz, chloroform-methanol-2.5 M ammonia (60:40:9) and the gangliosides were visualized with resorcinol reagent. (Svennerholm L., Biochim. Biophys. Acta 24, 604-614, 1957). The fractions which contained sialosyllactotetraosylceramid were pooled and evaporated to dryness.

The final purification of sialosyllactotetraosylceramid was achieved by preparative thin-layer chromatography. Ganglioside ( 5 $\mu$moles) was applied on a thin-layer plate (20x20 cm, 0,25 mm thick, Merck, AG., Darmstadt, West Germany), which was developed for 1 hour with chloroform-methanol-2.5 M ammonia at 21°C. After completed chromatography the plate was sprayed with 0,01% bromthymol blue in water and the sialosyllactotetraosylceramid band was scraped off. The ganglioside was eluted from the gel with chloroform-methanol-water (30:60:20, by volume) and one drop of 0,1 M pottassium hydroxide dissolved in methanol was added and the mixture was evaporated to dryness. The ganglioside was dissolved in an small volume of chloroform-methanol (2:1, by volume) and was kept in a tight tube C Klimax tube with a teflon screw cap) in the dark at +4°C.

Example 2

USE OF PURIFIED SIALOSYLLACTOTETRAOSYLCERAMID GANGLIOSIDE FOR THE PRODUCTION OF MONOCLONAL ANTIBODIES DIRECTED AGAINST THE SIALOSYLLACTOTETRAOSE ANTIGEN.

The purified sialosyllactotetraosylceramid ganglioside from Example 1 was adsorbed to acid-washed Salmonella Minnesota bacteria before immunization according to the principle, described by Young, W.W. and collaborators (J. Exp. Med. 150, 1008-1019, 1979).

Balb/c mice, 6-8 weeks old, were immunized intravenously with 5 $\mu$g of ganglioside, adsorbed to 50 $\mu$g acid-washed Salmonella Minnesota bacteria suspended in 100 $\mu$l of phosphate-buffered physiological saline, pH 7.4. A new immunization was performed after two weeks with the same dose of immunogen under identical conditions (booster dose).

Three days after the booster dose, the spleen cells taken from the immunized mice were isolated and fused with mouse Sp 2/0 myeloma cells. The resulting hybridomas were expanded, tested and cloned according to the protocol, devised by de St. Groth, S.F. and Scheidegger (J. Immunol. Methods, 1-21, 1980). The culture medium was collected from each microtiter well with growing hybridomas to determine whether the medium contained antibodies directed against sialosyllactotetraose. The determination was performed in an ELISA system according to the following procedure: The sialosyllactotetraosylceramide ganglioside was dissolved in methanol and 50 pmol was added to each well of a polyvinylmicrotiter plate. Upon evaporation of the methanol the wells were provided with 100 $\mu$l of hybridoma culture medium diluted with an equally large volume of 0,01 M phosphate buffered saline (0,14 M), pH 7,5. The incubation was carried out for 17 hours. Then 100 $\mu$l of peroxydase (HRP)-conjugated rabbit anti-mouse immunoglobulin diluted 1/200 in the above phosphate buffer, now provided with 1% bovine serum albumin was added to the well. The absorbance at 450 nm was determined in a Titertek microtiter scanner after incubation for 4 hours and addition of 100 $\mu$l of enzyme substrate to the well (0,1% ortophenyldiamin i citrate buffer, pH 4,5, mixed with 0,3% $H_2O_2$).

Hybrids were regarded as positive if the absorbance exceeded that of the mean medium blank by a 2-fold or more. Culture medium from positive hybrids was collected for further screeing (see below) whereas the positive hybrids were frozen in liquid nitrogen. Culture medium from hybrids positive against sialosyllactotetraosylceramide was tested against human lymphocytes for cross-reactivity. Human lymphocytes (HuLy-c) were isolated by gradient centrifugation of blood from five donors of all different ABH-blood groups and the cells were pooled. An ELISA-test on the immobilized cells was carried out. Hybrids that produced antibodies cross-reacting with HuLy-c were not further expanded. The ganglioside positive/HuLy-c negative hybrids were cloned by limited dilution and expanded. The isotype of each clone was determined by Mancinitechnique using isotype specific antimouse immunoglobulins.

The reactivity of the ganglioside positive/HuLy-c-negative clones were determined in a double antibody solid phase radioimmuno assay. The sialosyllactotetraosylceramid ganglioside and a large number of neutral glycolipids and other gangliosides were dissolved in methanol and pipetted into the wells of a microtiter plate and the methanol was evaporated. Upon incubation with the monoclonal antibodies against sialosyllactotetraosylceramid the amount of bound was detected with $^{125}$I-labelled anti-mouse immunoglobulin.

The specificity of the monoclonal antibodies were also determined by thin-layer chromatographic immunostaining of the ganglioside antigens.

The total gangloside fraction isolated from a number of different tumor tissues were separated on aluminia-backed high performance thin-layer plates (HPTLC) and incubated with the culture medium from the positive hybridomas, diluted with Tris buffered salin (pH 7,8) containing 1% bovine serum albumin. Bound monoclonal antibody was detected with $^{125}$I-labelled antimouse immunoglobulin which was exposed to X-ray film for 12-24 h.

The monoclonal antibodies examined have all reacted negatively against neutral glycolipids. The major part of the antibodies having been positive against the sialosyllactotetraosylceramid ganglioside have shown a vigorous reaction with gangliosides having the same carbohydrate structure but also containing fucose linked to N-acetylglucoseamine.

Example 3

DETERMINATION OF SIALOSYLLACTOTETRAOSE ANTIGEN I TUMOR TISSUED

The tumor antigen has been isolated both as a glycolipid as well as a glycoprotein. When the antigen has been isolated as a ganglioside, the same principle methos was used as described in Example 2, but the method has been adapted to small tissue samples used for analysis.

Detection of the gangliosideantigen

0,5 ml of water was added to about 0,1 g of tissue and the tissue was homogenized in a conical homogenizer with teflon pestle in an ice bath. After finished homogenization 1.6 ml of methanol and 0.8 ml of chloroform were added. After thorough mixing the tube was centrifuged att 100xg for 10 min. The clear supernatant was transferred to a small Kimax-tube with teflon cap. The sediment in the tube was suspended in 0.5 ml of water and then methanol and chloroform were added in the same volumes as used in the first extraction. Mixing and centrifugation were performed as before. The two supernatants were combined and water was added to give a final chloroform-methanol-water ratio 4:8:5.6 (by volume). Centrifugation was performed at 100xg for 5 min. The upper phase was transferred to a small round flask with ground neck and 0.5 ml chloroform and 1.0 ml water were added to the lower phase. Upon mixing 0.7 ml water was added and after renewed mixing centrifugation was performed at 100xG for 5 min. The upper phase was transferred to the round flask with ground neck and 0,1 volume of iso butanol added. The residue was evaporated to dryness with 0.1 M NaOH in methanol. The tube was left for 1 h at room temperature.

After finished transesterification 4 ml of chloroform and 1 ml of methanol were added and the content was poured into a small column with 1 g of Sephadex® G 25, packed in chloroform-methanol-water (60:30:4.5 by volume). The column was eluated with 15 ml chloroform-methanol-water (60:30:4.5). The eluate was slowly added to a column packed with 1.0 g of DEAE-Sepharose® (Pharmacia Fine Chemicals, Uppsala, Sweden) in acetate form. After rinsing the column with 10 ml of methanol, the monosialogangliosides were eluted with 10 ml of 0,02 M potassium acetate in methanol. After evaporation and redissolution in chloroform-methanol-water (65:25:4 by volume) the simple monosialogangliosides were separated from sialosyllactotetraosylceramid by elution with 15 column volumes of 65:25:4 (weight/volume), whereafter the complex ganglioside fraction was eluted with 10 volumes of chloroform-methanol-water (50:40:10 by volume).

An aliquot of the purified complex ganglioside fraction of tissue was dissolved in methanol and 50 $\mu$l of extract was added to one of the wells of a polyvinyl microtiter plate. Standard solutions of 1 to 25 pmol of pure sialosyllactotetraosylceramid in methanol were added to the other wells of the plate. The solvent was evaporated under a stream of nitrogen. To the unknown samples and standards there were added 10-100 $\mu$g of monoclonal antibody, specific for sialosyllactotetraose with fucose, if any, linked to said molecule, and produced as described i Example 2, dissolved in phosphat-buffered physiological saline, pH 7.4, as well as 1% bovine serum albumin (PBS). The samples were incubated for 1 h at room temperature, and then the well was washed 3 times with PBS solution. Then $^{125}$I-labelled antimouse-immunoglobulin was added and allowed to react for 3 hours. After repeated washing with PBS the wells with unknowns and standards were cut out from the microtiter plate and counted in a gamma counter. The concentration of sialosyllactotetraose antigen of the unknowns were calculated from the standard curve of pure sialosyllactotetraosylceramid.

The protein-linked form of the sialosyllactotetraose antigen was detected by means of immunological isotope and enzyme methods. 100 $\mu$l of the aqueous homogenate of tumor tissue were added to an incubation tube and then 100 $\mu$l of monoclonal antibody were added and mixing was carried out. The tube was scaled and incubated for 90 min. at room temperature and under slow shaking. To each tube there was added a polystyrene bead on which a thin layer of the sialosyllactotetraosylceramide ganglioside antigen had been applied. After the addition of the antigen bead the tube was tapped slightly in order to remove any air bubbles present on the plastic bead. Then the sample was incubated for 4 hours. The incubation solution was sucked off with a water aspirator and the beads were washed with 2x2 ml of phosphate-buffered physiological saline. The washing liquid was removed as careful as possible. Then 200 $\mu$l of $^{125}$I-labelled antimouse antibody were added and incubation was performed during the night at +4°C. The antibody solution was sucked off and the beads were washed with 4x2 ml of phosphate-buffered physiological saline. The washing liquid was removed carefully between each washing. The incubation tube with bead was placed in a gamma counter and the number of cpm of $^{125}$I was determined. At each analysis a blank (free from antigen) was analysed. Further, at least one positive sample (an aqueous extract from a tumor with known occurence of antigen) was always analysed. The inhibition was determined and from the first value it has been possible to make dilutions of the tumor extract in case the inhibition has been too big. The sialosyllactotetraose inhibition test gav a highfrequency of positivity in different forms of tumor varying between 35-80%. A certain activity of the water-soluble antigen was found in certain organs, such as the small intestine, the large intestine and pancreas. The sialosyllactotetraose antigen test in lipid extract (as ganglioside) gave, as a rule, higher specificity (no positive tests in normal tissue) as well as a larger number of positive tests on tumor tissue. In the present case the detection step is based upon an isotope-labelled second antibody in the immunological inhibition test.

Example 4

DETERMINATION OF SIALOSYLLACTOTETRAOSE ANTIGEN IN BLOOD SERUM FROM PATIENTS HAVING CARCINOMA

100 $\mu$l of patient serum were added to an incubation tube and then 100 $\mu$l of the monoclonal antibody were added, and mixing was performed. The tube was capped and incubated for 90 min. at room temperature under slow shaking. Then there was added to each tube a polystyrene bead coated with sialosyllactotetraosylceramid. Any air bubbles present were allowed to disapperar by careful tapping of the tube. The incubation was performed for additional 4 hours. After said time the incubation solution was sucked off with a water aspirator and the beads were washed twice with 2 ml Of phosphate-buffered physiological saline. The washing liquid was removed as careful as possible. Then 200 $\mu$l of $^{125}$I-antimouse antibody were added and incubated over the night at +4°C. After said time the antibody solution was sucked off and the beads were washed four times with 2 ml of phosphate-buffered physiological saline. The washing liquid was removed carefully between each washing. The incubation tube with its polystyrenebead was counted in a gamma counter and the number of counts was determined.

Example 5

DETERMINATION OF SIALOSYLLACTOTETRAOSYLCERAMID IN BLOOD SERUM FROM PATIENTS WITH CARCINOMA

1 ml of blood serum diluted with 0,5 ml of physiological saline was added dropwise to 4 ml of methanol in a small Kimax tube under continous mixing. Then 2 ml of chloroform were added and the sample was thoroughly mixed and centrifugated at 800xG for 10 min. The clear centrifugate was transferred to a small round flask and was evaporated to dryness. It was dissolved in 5 ml of chloroform and was applied on 1 g silica gel column (Kieselgel 60, 230-400 mesh, Merck AG, Darmstadt, West Germany). The column was eluated with 10 ml of chloroform and 15 ml of chloroform-methanol-water (65:25:4, by volume) and then the ganglioside antigen was eluated with 10 ml of chloroform-methanol-water (50:40:10). The last eluate was added slowly to a column of 1 g DEAE-Sepharose, which then was eluated with 10 ml of methanol. Complex monosialogangliosides were the eluated with 10 ml of 0,02 M potassium acetate in methanol. The salt was removed by dialysis against distilled water and the ganglioside fraction was dissolved in methanol.

The concentration of sialosyllactotetraosylceramid was determined with a double solid phase antibody method, in which the ganglioside was adsorbed to the solid phase and determined with monoclonal antibody and antimouse-immunoglobulin. 20 pmol of pure sialosyllactotetraosylceramid and samples of pure monosialoganglioside fraction from serum were dissolved in methanol and serial dilutions of them (1/2-1/2048) were pipetted in the wells of polyvinyl microtiter plates (Flow Laboratories art.nr. 77-173-05) and were evaporated. The wells were blocket with 20 $\mu$l of 1% bovine serum albumin (BSA) in phosphate-buffered physiological saline (PBS) and then 50 $\mu$l of monoclonal antibody against the antigen, diluted in 1% BSA in PBS, were added. After incubation for 4 hours, the wells were rinsed with 3x200 $\mu$l of PBS.

After the rinsing fixed monoclonal antibody was measured by addition of antimouse im-munoglobulin labelled with $^{125}$I or with peroxydase or $\beta$-galactosidase. Measurement of the concentration of the other antibody was performed in a gamma counter or after addition of enzyme substrate (ortophenyl diamin or methylumbelliferyl-$\beta$-

galactoside in citrate buffer, pH 4.2, and reading of the fluorescens in a spectrofluorimeter after 30 min). The concentration of sialosyllactotetraosylceramid was calculated from standard samples of the purified ganglioside. Under the conditions specified above, antigen serum was only detected in patients with adenocarcinoma.

**Claims**

1. An antibody having specificity for:
   (i) NeuNAcα-3Galβ-3GlcNAcβ-3Galβ-4Glc,
   or
   (ii) both NeuNAcα-3Galβ-3GlcNAcβ-3Galβ-4Glc and
   NeuNAcα-3Galβ-3GlcNAcβ-3Galβ-4Glc,

$$4$$
$$|$$
Fucα

   or
   (iii) the member of (i) or (ii) glycosidically bound to a lipid or protein moiety at the terminal glucose.
   for use in the diagnosis in vivo and therapeutic treatment of diseases related to tumor.

2. The antibody of claim 1 *characterized* by being produced by monoclonal technique.

3. The antibody of any of claims 1-2 *characterized* by having specificity for the carbohydrate structures given in (ii) and for said structures bound as set forth in (iii).

4. A method for the detection of carcinomas in a patient comprising the steps of:
   (a) contacting a tissue or body fluid sample, which has been obtained from said patient, with an antibody being specific for a tumour antigen so that said antibody can bind immunologically to the tumour antigen; and
   (b) detecting and taking the occurrence of said binding as an indication of said patient having a carcinoma,
   *characterized* by the antibody used having specificity for:
   (i) NeuNAcα-3Galβ-3GlcNAcβ-3Galβ-4Glc,
   or
   (ii) both NeuNAcα-3Galβ-3GlcNAcβ-3Galβ-4Glc and
   NeuNAcα-3Galβ-3GlcNAcβ-3Galβ-4Glc,

$$4$$
$$|$$
Fucα

   or
   (iii) the members of (i) or (ii) glycosidically bound to a lipid or protein moiety at the terminal glucose.

5. The method of claim 4, characterized by the antibody used having specificity for the carbohydrate structures given in (ii) and for said structures bound as set forth in (iii).

6. The method of claim 4 or 5, characterized by said sample being serum, blood, urine or saliva.

7. The method of claim 4, 5, or 6 *characterized* by said binding being detected by the use of an antibody labeled with an enzyme, a radioactive isotope or a fluorochrome.

**Revendications**

1. Anticorps ayant une spécificité pour :
   (i) NeuNAcα-3Galβ-3GlcNAcβ-3Galβ-4Glc,
   ou
   (ii) à la fois le NeuNAcα-3Galβ-3GlcNAcβ-3Galβ-4Glc et le NeuNAcα-3Galβ-3GlcNacβ-3Galβ-4Glc,

$$4$$
$$|$$
Fucα

   ou
   (iii) le composé du paragraphe (i) ou (ii) fixé par une liaison glycosidique à un groupement lipidique ou protéique au niveau du glucose terminal,
   destiné à être utilisé dans le diagnostic in vivo et le traitement thérapeutique de maladies liées à la présence d'une tumeur.

2. Anticorps suivant la revendication 1, caractérisé en ce qu'il est produit par la technique de production d'anticorps monoclonaux.

3. Anticorps suivant l'une quelconque des revendications 1 et 2, caractérisé en ce qu'il possède une spécificité pour les structures glucidiques mentionnées en (ii) et pour lesdites structures liées de la manière indiquée en (iii).

4. Méthode de détection de carcinomes chez un patient, comprenant les étapes :
   (a) de mise en contact d'un échantillon de tissu ou de liquide biologique, qui a été obtenu chez ledit patient, avec un anticorps spécifique d'un antigène tumoral de sorte que ledit anticorps puisse présenter une liaison immunologique avec l'antigène tumoral ;
   et
   (b) de détection et de considération de la présence de ladite liaison comme une indication de la présence d'un carcinome chez ledit patient,
   caractérisée en ce que l'anticorps utilisé possède une spécificité pour :
   (i) NeuNAcα-3Galβ-3GlcNAcβ-3Galβ-4Glc, ou
   (ii) à la fois le NeuNAcα-3Galβ-3GlcNAcβ-3Galβ-4Glc et le NeuNAcα–3Galβ-3GlcNacβ-3Galβ-4Glc,

$$\overset{\textbf{4}}{\underset{\text{Fuc}\alpha}{|}}$$

   ou
   (iii) les composés du paragraphe (i) ou (ii) fixés par une liaison glycosidique à un groupement lipidique ou protéique au niveau du glucose terminal.

5. Méthode suivant la revendication 4, caractérisée en ce que l'anticorps utilisé possède une spécificité pour les structures glucidiques mentionnées en (ii) et pour lesdites structures liées de la manière indiquée en (iii).

6. Méthode suivant la revendication 4 ou 5, caractérisée en ce que l'échantillon est un échantillon de sérum, de sang, d'urine ou de salive.

7. Méthode suivant la revendication 4, 5 ou 6, caractérisée en ce que la liaison est détectée au moyen d'un anticorps marqué avec un enzyme, un isotope radioactif ou un fluorochrome.

**Patentansprüche**

1. Antikörper mit Spezifität für:
   (i) NeuNAcα-3Galβ-3GlcNAcβ-3Galβ-4Glc, oder
   (ii) sowohl NeuNAcα-3Galβ-3GlcNAcβ-3Galβ-4Glc als
   auch NeuNAcα-3Galβ-3GlcNAcβ-3Galβ-4Glc,

$$\overset{\textbf{4}}{\underset{\text{Fuc}\alpha}{|}}$$

   oder
   (iii) die Verbindung nach (i) oder (ii) in glycosidischer Bindung an einen Lipid- oder Proteinrest an der endständigen Glucose,
   zur Verwendung bei der in vivo-Diagnose und therapeutischen Behandlungen von Krankheiten im Zusammenhang mit Tumor.

2. Antikörper nach Anspruch t, dadurch **gekennzeichnet,** daß er nach einer monoklonalen Technik hergestellt ist.

3. Antikörper nach einem der Ansprüche 1 bis 2, dadurch **gekennzeichnet,** daß er für die Kohlenhydratstrukturen, die in (ii) angegeben sind und für die gemäß (iii) gebundenen Strukturen spezifisch ist.

4. Verfahren zur Detektion von Karzinomen bei einem Patienten, umfassend die Stufen:
   (a) Kontaktieren einer Gewebs- oder Körperflüssigkeitsprobe, die von den Patienten erhalten worden ist, mit einem Antikörper, der für ein Tumorantigen spezifisch ist, so daß der Antikörper immunologisch an das Tumorantigen binden kann;
   und
   (b) Detektieren und Werten des Vorkommens der Bindung als Anzeichen dafür, daß der Patient ein Karzinom hat,
   dadurch **gekennzeichnet,** daß der verwendete Antikörper Spezifität für:
   (i) NeuNAcα-3Galβ-3GlcNAcβ-3Galβ-4Glc, oder
   (ii) sowohl NeuNAcα-3Galβ-3GlcNAcβ-3Galβ-4Glc als
   auch NeuNAcα-3Galβ-3GlcNAcβ-3Galβ-4Glc,

$$\overset{\textbf{4}}{\underset{\text{Fuc}\alpha}{|}}$$

   oder
   (iii) die Verbindungen nach (i) oder (ii) in glycosidischer Bindung an einen Lipid- oder Proteinrest an der endständigen Glucose,
   besitzt.

5. Verfahren nach Anspruch 4, dadurch **gekennzeichnet,** daß der verwendete Antikörper Spezifität für die in (ii) angegebenen Kohlenhydratstrukturen und für die gemäß (iii) gebundenen Strukturen besitzt.

6. Verfahren nach Anspruch 4 oder 5, dadurch **gekennzeichnet,** daß die Probe Serum, Blut, Urin oder Speichel ist.

7. Verfahren nach Anspruch 4, 5 oder 6, dadurch **gekennzeichnet,** daß die Bindung durch Verwendung eines mit einem Enzym, einem radioaktiven Isotop oder einem Fluorochrom markierten Antikörpers detektiert wird.